# EUROPÄISCHE PATENTSCHRIFT

(11) **EP 3 620 066 B1**
(45) Veröffentlichungstag und Bekanntmachung des Hinweises auf die Patenterteilung: **08.05.2024**
(21) Anmeldenummer: 19020311.7
(22) Anmeldetag: 26.04.2019
(51) Int. Cl.: A23L 33/105, A61K 36/00

(54) **NAHRUNGSMITTELZUSAMMENSETZUNG IN KAPSELFORM**
FOOD COMPOSITION IN CAPSULE FORM
COMPOSITION ALIMENTAIRE SOUS FORME DE GÉLULE

(30) Priorität: 27.04.2018 DE 102018110181; 28.06.2018 DE 102018115563
(43) Veröffentlichungstag der Anmeldung: 11.03.2020
(73) Patentinhaber: Zulauf, Edeltraud Antonie, 59929 Brilon (DE)
(72) Erfinder: Zulauf, Edeltraud Antonie, 59929 Brilon (DE)
(74) Vertreter: Ostermann, Thomas

(56) Entgegenhaltungen:
- WO-A1-99/55351
- RU-C2- 2 494 635
- US-A1- 2009 214 680
- US-A1- 2017 028 004
- US-B2- 7 056 539

## Beschreibung

Die Erfindung betrifft eine Nahrungsmittelzusammensetzung zur Verbesserung von Entwässerung und/oder eines Energiestoffwechsels eines Fettsäurestoffwechsel von Menschen.

Aus der DE 10 2010 036 451 B4 ist eine Nahrungsmittelzusammensetzung zur Reduzierung von Körpergewicht bekannt. Hierzu unterstützt eine erste Komponente die Freisetzung von Fettsäuren in einem Organismus. Eine andere Komponente bewirkt eine verringerte Zuckeraufnahme im Darm. Die beiden Komponenten sollen gleichzeitig die Fettsäurefreisetzung erhöhen, während der Insulinspiegel gesenkt wird.

Aus der RU 2 494 635 C2 ist ein Schlankheitstee enthaltend Löwenzahn, Schachtelhalmen und Brennnessel bekannt. Die Bestandteile sind nicht in Form von Extrakten ausgeführt. Die Teemischungen weisen keine Zusätze auf.

Aus der US 2009/214680 A1 ist eine Nahrungsmittelzusammensetzung zur Erreichung eines Gewichtsverlustes bekannt, die grüne Teeextrakte, Rhodiola Rosea-Teeblätter und dergleichen sowie Calcium enthält. Die Nahrungsmittelzusammensetzung enthält darüber hinaus Proteine, Ginseng, Koffeine und dergleichen.

Aufgabe der vorliegenden Erfindung ist es, eine Zusammensetzung zur Verfügung zu stellen, mittels derer eine Gewichtsreduzierung ohne gesundheitsschädliche Nebenwirkungen gewährleistet ist, wobei eine langfristige nutritive Ergänzung erlaubt ist.

Diese Aufgabe weist die Merkmale des Anspruchs 1 auf.

Komponente a) unterstützt die Entwässerung in einem Organismus eines Menschen oder eines Tieres. Komponente b) unterstützt den Energiestoffwechsel in dem Organismus des Menschen bzw. Tieres, Komponente c) unterstützt den Fettsäurestoffwechsel in dem Organismus des Menschen bzw. Tieres. Vorzugsweise werden die Komponenten a), b), c) über den Tag verteilt zu sich genommen. Vorteilhaft ermöglicht die Erfindung eine umfassende Verbesserung des Stoffwechsels, wobei eine Gewichtsreduktion einhergeht. Besonders gute Ergebnisse werden erzielt, wenn die Komponenten a), b), c) an Tagen eingenommen werden, an denen die einnehmende Person eine körperliche Tätigkeit ausübt, beispielsweise Trainingseinheiten einer Länge von ca. einer Stunde an zwei bis vier Tagen pro Woche.

Unter einem Extrakt entsprechend den Komponenten a), b), c) werden im Sinne der vorliegenden Erfindung Auszüge aus Pflanzen verstanden. Der Extrakt liegt vorzugsweise in fester, beispielsweise pulverförmiger, Form vor.

Der Extrakt kann durch Zerkleinern, Pressen, Extrahieren, Zentrifugieren, Filtrieren und/oder Säulentrennung der Pflanzen hergestellt werden. Die höchste Konzentration der Wirkstoffe wird durch Extrahieren mittels eines Lösungsmittels erreicht. Durch Abdestillieren des Lösungsmittels kann beispielsweise das Extrakt gewonnen werden. Als organische Lösungsmittel können beispielsweise Alkohole oder dergleichen eingesetzt werden. Beispielsweise kann die Pflanze in der Flüssigkeit (Alkohol oder Wasser) eingelegt und darin erhitzt werden. Dabei gehen die Wirkstoffe der Pflanze auf das Extraktionsmittel über. Danach wird das Extraktionsmittel verdampft, wodurch ausschließlich die Wirkstoffe als Trockenextrakt (Extrakt-Pulver) übrigbleiben.

Die Komponente a) wird als ein Extrakt einer Mischung aus Rhodiola Rosea-Tee, Brennnesselblättern und Birkenblättern eingesetzt, wobei die Bestandteile des Extraktes gemischt werden. Die Komponente a) dient zur Verbesserung der Entwässerung und sollte einmal am Tag mit vorzugsweise 0,5 L Wasser zu sich genommen werden.

Die Komponente b) wird als ein Extrakt einer Mischung aus Brennnesselblättern, Wacholder, Matetee und Schachtelhalmen eingesetzt. Diese Komponente dient vorzugsweise zur Verbesserung des Energiestoffwechsels und sollte einmal täglich mit vorzugsweise 0,5 L Wasser eingenommen werden.

Die Komponente c) wird als ein Extrakt einer Mischung aus Löwenzahnblättern und Artischocken eingesetzt, was zur Fettsäurestoffwechselsteigerung dient. Vorzugsweise soll dieses Extrakt einmal täglich mit vorzugsweise 0,5 L Wasser eingenommen werden.

Die Komponente a) weist einen Anteil Calcium auf.

Die Komponente b) weist einen Anteil Magnesium auf.

Die Komponente c) weist einen Anteil Zink und vorzugsweise einen Anteil Cynarin auf.

Die vorliegende Erfindung betrifft weiterhin ein Kit-of-Parts, das getrennt voneinander umfasst
A) eine Zusammensetzung, die Komponente a) enthält, und
B) eine Zusammensetzung, die Komponente b) enthält, und
C) eine Zusammensetzung, die Komponente c) enthält.

Im Sinne der Erfindung können die Zusammensetzung A), die Zusammensetzung B) und die Zusammensetzung C) gleich oder zeitgleich versetzt verabreicht bzw. eingenommen werden. Zusammensetzung A besteht aus der Komponente a). Die Zusammensetzung B) besteht aus der Komponente b).

Die Zusammensetzung c) besteht aus der Komponente c). Vorzugsweise liegen die Zusammensetzungen A), B) und C) in Gelatinekapselform vor.

Die erfindungsgemäße Nahrungsmittelzusammensetzung kann alleine oder als Bestandteil eines Kit-of-Parts verabreicht werden. Wenn die erfindungsgemäße Nahrungsmittelzusammensetzung insbesondere als Mittel zur Körpergewichtsreduktion verwendet wird, ist die Verabreichung als Bestandteil eines Kit-of-Parts vorteilhaft, dessen Bestandteile über den Tag verteilt, beispielsweise morgens, mittags und abends, verabreicht werden, um eine permanente Versorgung und/oder Wirkung über den Tag zu erzielen.

Die erfindungsgemäße Nahrungsmittelzusammensetzung wird derart pro Tag verabreicht, dass als wirksame Bestandteile
- 100 mg Brennnesselblatt,
- 100 mg Rhodiola Rosea-Tee,
- 100 mg Birkenblätter,
- 100 mg Wacholder,
- 100 mg Matetee,
- 100 Schachtelhalme,
- 220 mg Löwenzahnblätter,
- 220 mg Artischocken
enthält.

Die Erfindung wird nachfolgend anhand von Beispielen detaillierter beschrieben. In den Beispielen werden Extrakte eingesetzt, die beispielsweise in Pulverform vorliegen. Es versteht sich, dass die Darreichungsformen der erfindungsgemäßen Nahrungsmittelzusammensetzung harte oder weiche Kapseln, wie beispielsweise Gelatinekapseln sind. Diese Gelatinekapseln enthalten übliche zur Formulierung der jeweiligen Darreichungsform eingesetzten Träger-, Verarbeitungs-, Hilfs- und/oder Zusatzstoffe.

### Beispiel 1

In Beispiel 1 ist eine Komponente a) der Zusammensetzung gemäß Anspruch 1 zur Entwässerung dargestellt:

| **Bestandteil** | **Menge** |
|---|---|
| Brennesselblätter | 100 mg |
| Rhodiola Rosea | 100 mg |
| Birkenblatt | 100 mg |
| Calcium | 120 mg |

Das Rhodiola Rosea, die Birkenblätter und die Brennnesselblätter können als Extrakte vorliegen. Als Trennmittel können Magnesiumsalze der Speisefettsäuren, Siliciumdioxid eingesetzt werden.

Das Calcium dient zu einer verbesserten Funktion der Verdauungsenzyme. Die Bestandteile des Beispiels 1 werden in einer Kapsel einmal täglich mit beispielsweise 0,5 L Wasser eingenommen.

### Beispiel 2

In Beispiel 2 ist eine erfindungsgemäße Komponente b) der Nahrungsmittelzusammensetzung nach Anspruch 1. zur Förderung des Energiestoffwechsels dargestellt:

| **Bestandteil** | **Menge** |
|---|---|
| Brennnesselblätter | 100 mg |
| Wacholderblätter | 100 mg |
| Matetee | 100 mg |
| Schachtelhalm | 100 mg |
| Magnesium | 55 mg |

Die Brennnesselblätter, die Wacholderblätter, der Matetee und der Schachtelhalm können als Extrakte vorliegen. Magnesium trägt zu einem verbesserten Energiestoffwechsel bei.

Als Trennmittel sind Magnesiumsalze der Speisefettsäuren und Siliciumdioxid vorgesehen. Die Zusammensetzung gemäß Beispiel 2 soll einmal täglich in einer Kapsel mit vorzugsweise 0,5 L Wasser eingenommen werden.

### Beispiel 3

In Beispiel 3 ist eine Komponente c) der Nahrungsmittelzusammensetzung gemäß Anspruch 1 dargestellt.

| **Bestandteil** | **Menge** |
|---|---|
| Löwenzahnblätter | 220 mg |
| Artischocken | 220 mg |
| Cynarin | 11 mg |
| Zink | 1,5 mg |

Die Löwenzahnblätter und die Artischocken können als Extrakte vorliegen. Als Trennmittel sind Magnesiumsalze der Speisefettsäuren und Siliciumdioxid vorgesehen. Die Zusammensetzung gemäß Beispiel 3 soll einmal täglich in Form einer Kapsel mit beispielsweise 0,5 L Wasser eingenommen werden.

Das Zink fördert einen verbesserten Fettsäurestoffwechsel.

Die vorgenannten Extrakte aus Blättern und/oder Pulver weisen im Vergleich zu einer Teemischung eine erhöhte Wirkung auf.

Die in den Beispielen 1, 2 und 3 genannten Nahrungsmittelzusammensetzungen, die in Kapselform vorliegen, werden jeweils zusammen mit einem halben Liter Flüssigkeit (Wasser) eingenommen. Die jeweils in einer einzigen Kapsel vorliegende Nahrungsmittelzusammensetzung kann über den Tag verteilt mit jeweils einem halben Liter Wasser oder auch unmittelbar nacheinander eingenommen werden, wobei allerdings dann 1,5 Liter Wasser getrunken werden sollte. Vorzugsweise sollen pro Tag jeweils eine Kapsel gemäß Beispiel 1, 2 und 3 eingenommen werden.

Weiterhin beschrieben, aber nicht Teil der vorliegende Erfindung sind folgende Teemischungen zubereitet aus Blättern.

Entwässerungstee:

| | |
|---|---|
| Folia Betulae | 20 Gew.% |
| Folia Melissae | 20 Gew.% |
| Rad. Liquiritiae | 20 Gew.% |
| Herba Solidaginis | 20 Gew.% |
| Folia Urticae | 20 Gew.% |

Fettverbrennungstee:

| | |
|---|---|
| Fructus Anisi | 10 Gew.% |
| Fructus Carvi | 10 Gew.% |
| Herba Agrimoniae | 10 Gew.% |
| Herba Taraxaci | 35 Gew.% |
| Folia Cynarae | 35 Gew.% |

Teemischung zur Verbesserung des Fettsäurestoffwechsels

| | |
|---|---|
| Fructus Juniperi | 4/88 Gew.% |
| Folia Menthae pip. | 4/88 Gew.% |
| Herba Taraxaci sine Rad. | 16/88 Gew.% |
| Folia Melissae | 16/88 Gew.% |
| Grüner Tee | 8188 Gew.% |
| Rotbusch Tee | 4/88 Gew.% |
| Mate grün | 4/88 Gew.% |
| Folia Urticae | 16/88 Gew.% |
| Herba Equiseti | 16/88 Gew.%. |

Die oben bezeichneten Teemischungen (Entwässerungstee, Fettverbrennungstee, Teemischung zur Verbesserung des Fettsäurestoffwechsels) werden folgendermaßen zubereitet. Die jeweiligen Teemischungen werden mit sprudelnd kochendem Wasser von jeweils 0,5 l aufgegossen. Die Ziehzeit beträgt mindestens 15 Minuten, vorzugsweise 15 bis 18 Minuten, vorzugsweise 15 Minuten. Die Teemischungen werden jeweils in einem gesonderten Behälter enthaltend 0,5 I sprudelnd kochendes Wasser aufgegossen. Die jeweiligen Teemischungen können über den Tag verteilt oder unmittelbar nacheinander eingenommen werden. Insgesamt nimmt die Person 1,5 l Flüssigkeit (Teemischungen) am Tag zu sich, wobei sie 0,5 l Entwässerungstee, 0,5 l Fettverbrennungstee und 0,5 l Teemischung zu Verbesserung des Fettsäurestoffwechsels tageweise zu sich nimmt.

Alternativ kann mindestens ein Bestandteil der Teemischungen eine Abweichung von der genannten Gew. %-Angabe von maximal +/- 10 % aufweisen.

## Patentansprüche

1. Nahrungsmittelzusammensetzung, enthaltend
a) ein Extrakt aus 100 mg Brennnesselblättern, 100 mg Rhodiola Rosea, 100 mg Birkenblättern und 120 mg Calcium und
b) ein Extrakt aus 100 mg Brennnesselblättern, 100 mg Wacholderblättern, 100 mg Matetee, 100 mg Schachtelhalm und 55 mg Magnesium und
c) ein Extrakt aus 220 mg Löwenzahnblättern, 220 mg Artischocken, 11 mg Cynarin und 1,5 mg Zink,
wobei die Komponenten in Form von Kapseln und mit vorzugsweise 0,5 L Wasser einnehmbar sind.

2. Kit-of-Parts, umfassend getrennt voneinander
A) eine Zusammensetzung, die Komponente a) enthält und
B) eine Zusammensetzung, die Komponente b) und
C) eine Zusammensetzung, die Komponente c) enthält.

## Claims

1. A food composition, including
a) an extract from 100 mg of nettle leaves, 100 mg of Rhodiola rosea, 100 mg of birch leaves, and 120 mg of calcium and
b) an extract from 100 mg of nettle leaves, 100 mg of juniper leaves, 100 mg of mate tea, 100 mg of horsetail, and 55 mg of magnesium and
c) an extract from 220 mg of dandelion leaves, 220 mg of artichoke, 11 mg of cynarine, and 1.5 mg of zinc,
wherein the components are consumable in the form of capsules and preferably with 0.5 l of water.

2. A kit of parts, comprising, separately from each other,
A) a composition, including the components a) and
B) a composition, including the components b) and
C) a composition, including the components c).

## Revendications

1. Composition alimentaire, incluant
a) un extrait de 100 mg de feuilles d'ortie, 100 mg de Rhodiola Rosea, 100 mg de feuilles de bouleau et 120 mg de calcium et
b) un extrait de 100 mg de feuilles d'ortie, 100 mg de feuilles de Genévrier, 100 mg de maté, 100 mg de prêle et 55 mg de magnésium et
c) un extrait de 220 mg de feuilles de pissenlit, 220 mg d'artichaut, 11 mg d'acide dicaféylquinique et 1,5 mg de zinc,
les composants pouvant être consommés en forme de gélules et de préférence avec 0,5 l de l'eau.

2. Ensemble d'éléments, comprenant séparément les uns des autres,
A) une composition, qui inclut les composants a) et
B) une composition, qui inclut les composants b) et
C) une composition, qui inclut les composants c).
